# EUROPÄISCHE PATENTSCHRIFT

(11) **EP 1 871 340 B1**
(45) Veröffentlichungstag und Bekanntmachung des Hinweises auf die Patenterteilung: **29.07.2009**
(21) Anmeldenummer: 06723881.6
(22) Anmeldetag: 31.03.2006
(51) Int. Cl.: A61K 9/08, A61K 33/00, A61K 36/00

(54) **REIZLINDERNDES HUSTENMITTEL ENTHALTEND EXTRAKTE AUS ISLÄNDISCHEM MOOS UND MALVE SOWIE ZINKVERBINDUNGEN**
ANTI-IRRITATING COUGH MIXTURE CONTAINING EXTRACTS FROM ISLAND MOSS AND MALLOW AS WELL AS ZINC DERIVATIVES
MEDICAMENT ANTITUSSIF ET SEDATIF CONTENANT DES EXTRAITS DE MOUSSE D'ISLANDE ET DE MAUVE ET EN PLUS DES DERIVES DE ZINC

(30) Priorität: 18.04.2005 DE 102005017923
(43) Veröffentlichungstag der Anmeldung: 02.01.2008
(73) Patentinhaber: Maria Clementine Martin Klosterfrau Vertriebsgesellschaft mbH, 50670 Köln (DE)
(72) Erfinder: VESTWEBER, Anna-Maria, 51399 Burscheid (DE); MIETHING, Holger, 12107 Berlin (DE)
(74) Vertreter: Gesthuysen, Michael
(86) Internationale Anmeldenummer: PCT/EP2006/002918
(87) Internationale Veröffentlichungsnummer: WO 2006/111258

(56) Entgegenhaltungen:
- AT-B- 162 431
- DE-U1-7202004 016 07
- FR-A- 2 157 676
- "External use agent for removing freckles and spots from skin - comprises kojic acid (deriv.) and one or more plant extracts, e.g. from iceland moss, orris root, oak bark, gardenia, mallow, avens, citrus unshiu peel, grape, cornflower, etc" WPI WORLD PATENT INFORMATION DERWENT, DERWENT, GB, Bd. 14, Nr. 95 PA - SANSWI SEIYAKU PN - JP7025762 A, 27. Januar 1995 (1995-01-27), XP002045141
- BRAUN-H. FROHNE-D.: "Heilpflanzen-Lexikon für Ärzte und Apotheker" 1987, GUSTAV FISCHER VERLAG , STUTTGART, NEW YORK , XP002392235 Seite 56 - Seite 57 Seite 152 - Seite 153

## Beschreibung

Die vorliegende Erfindung betrifft eine pharmazeutische Zubereitung in Form einer flüssigen Dosierung, insbesondere zur Verwendung als reizlinderndes Hustenmittel, wie Hustensaft, auf Basis einer Kombination von Schleimdrogen bzw. deren Extrakten sowie die medizinische bzw. therapeutische Verwendung dieser pharmazeutischen Zubereitung.

Insbesondere betrifft die vorliegende Erfindung eine pharmazeutische Zubereitung in Form einer flüssigen Dosierung, insbesondere ein Hustenmittel, auf Basis einer Kombination von Isländischem Moos (*Lichen islandicus*) und Malve (*Malva sylvestris L.* bzw. *Malva neglecta WALLR.*), insbesondere deren Extrakten, gegebenenfalls in Kombination mit mindestens einer Zinkverbindung, sowie die medizinische bzw. therapeutische Verwendung dieser pharmazeutischen Zubereitung.

Weiterhin betrifft die vorliegende Erfindung die Verwendung einer Kombination von Isländischem Moos und Malve, gegebenenfalls zusammen mit mindestens einer Zinkverbindung, zu Zwecken der prophylaktischen oder therapeutischen Behandlung verschiedener Erkrankungen.

Insbesondere bei Infekten der Atemwege können äußere Reize, wie schlechte oder trockene Atemluft, die bereits angegriffene Hals- und Rachenschleimhaut austrocknen. Hustenreiz und Heiserkeit sind die Folge.

Hier kommen im allgemeinen sogenannte Hustenmittel zum Einsatz, wobei im allgemeinen zwischen hustenstillenden oder hustenhemmenden Hustenmitteln, sogenannten Antitussiva, einerseits und hustenbildenden bzw. auswurffördernden Hustenmitteln, sogenannten Expektorantien, andererseits unterschieden wird.

Während Antitussiva (z. B. Morphinderivate, wie z. B. Codein und dessen natürliche und synthetische Derivate, wie Dihydrocodein, Hydrocodon etc.) über eine Hemmung der reflektorischen Erregbarkeit des Hustenzentrums in der Medulla oblongata wirken und somit sozusagen das Hustenzentrum dämpfen bzw. den Hustenreflex hemmen, verstärken und/oder beschleunigen die Expektorantien (z. B. Ammoniumchlorid, Kaliumiodatum, ätherische Öle etc.) dagegen die physiologische Expektoration, wobei entweder die sekretolytische oder die sekretomotorische Wirkung überwiegt.

Die vorgenannten Substanzen haben zum Teil aber schwerwiegende Nebenwirkungen: Antitussiva auf Basis von Morphinderivaten können neben ihrer hustenhemmenden Wirkung auch analgetisch und/oder atemdepressiv wirken und zudem in unterschiedlichem Maße suchterzeugend sein. Auch einige der vorgenannten Expektorantien haben schwerwiegende Nebenwirkungen: So birgt die Anwendung von Ammoniumchlorid als Expektorans die Gefahr einer metabolischen Azidose, und die Verwendung des Expektorans Kaliumiodatum kann Iodismus auslösen.

Folglich besteht ein Bedarf an Hustenmitteln, welche die zuvor genannten Nachteile der Hustenmittel des Standes der Technik zumindest weitgehend vermeiden oder aber zumindest abschwächen.

Der vorliegenden Erfindung liegt daher die Aufgabe zugrunde, eine pharmazeutische Zubereitung bereitzustellen, welche sich zur effizienten Behandlung insbesondere von Husten eignet und die zuvor geschilderten Nachteile des Standes der Technik zumindest weitgehend vermeidet oder aber zumindest abschwächt. Eine solche pharmazeutische Zubereitung sollte gleichermaßen hustenreizlindernd wirken und insbesondere bei sogenanntem trockenem Reizhusten anwendbar sein.

AT 162 431, FR 2 157 676, WPI WORLD PATENT INFORMATION DERWENT, DERWENT, GB, Bd. 14, Nr. 95 PA - SANSWI SEIYAKU PN - JP7025762 A, 27. Januar 1995 (1995-01-27) offenbaren pharmazeutische Zubereitungen, die eine Kombination von Isländisch Moos und Malve auf einem pharmakologisch unbedenklichen Träger enthalten.

Die Anmelderin hat nun überraschenderweise herausgefunden, daß das zuvor geschilderte Problem dadurch gelöst werden kann, daß man eine Kombination von Isländischem Moos, Malve und einer Zinkverbindung in einer pharmazeutischen Zubereitung in Form einer flüssigen Dosierung formuliert. Eine solche pharmazeutische Zubereitung bzw. Zusammensetzung eignet sich insbesondere zur Verwendung als Hustenmittel, aber darüber hinaus auch für eine Reihe weiterer Indikationen, wie nachfolgend noch beschrieben.

Gegenstand der vorliegenden Erfindung ist somit eine pharmazeutische Zubereitung in Form einer flüssigen Dosierung, insbesondere ein reizlinderndes

Hustenmittel, welche in pharmazeutisch wirksamen Mengen eine Kombination von Isländischem Moos (*Lichen islandicus*) Malve (Malva sylvestris *L.* und *Malva neglecta WALLR.*) und einer Zinkvebindung zusammen mit einem pharmakologisch unbedenklichen Träger enthält.

Die pharmazeutische Zubereitung nach der vorliegenden Erfindung kann zur Behandlung verschiedener Erkrankungen eingesetzt werden, insbesondere von Husten und Hustenreiz, insbesondere bei trockenem Reizhusten, darüber hinaus aber auch bei Schleimhautreizungen und Schleimhautläsionen im Mund- und Rachenraum, Austrocknungen der Schleimhäute im Bereich des Mund- und Rachenraums, insbesondere infolge von Infekten, bei Heiserkeit sowie bei Bronchialkatharren und Bronchialasthma.

Durch die Verwendung natürlich basierter Wirkstoffe auf Basis der synergistischen Kombination der beiden Schleimdrogen Isländisches Moos einerseits und Malve andererseits werden die mit synthetischen Wirkstoffen auftretenden Nebenwirkungen wirksam verhindert.

Der Begriff "pharmazeutische Zubereitung" - synonym bisweilen auch als "pharmazeutische Zusammensetzung", "pharmazeutische Kombination" etc. bezeichnet - ist im Rahmen der vorliegenden Erfindung sehr weit zu verstehen und umfaßt jede Art von möglicher pharmazeutischer Zubereitung, Zusammensetzung oder Kombination, insbesondere Arzneimittel bzw. Pharmaka als solche, aber auch sogenannte Medizinprodukte, homöopathische Mittel etc.

Eine Besonderheit der vorliegenden Erfindung muß in der synergistischen Kombination der beiden Schleimdrogen Isländisches Moos (*Lichen islandicus*) einerseits und Malve (*Malva sylvestris L.* und *Malva neglecta WALLR.*) andererseits gesehen werden. Denn - wie die Anmelderin herausgefunden hat - wirkt eine derartige Kombination der beiden vorgenannten Schleimdrogen in synergistischer Weise, d. h. über die Wirkung der einzelnen Schleimdrogen hinausgehend.

Die beiden Schleimdrogen Isländisches Moos einerseits und Malve andererseits bzw. deren Extrakte sind dem Fachmann als solche hinlänglich bekannt:
Schleimdrogen werden wegen ihrer antiphlogistischen Eigenschaften äußerlich zur Behandlung von Furunkeln, Geschwüren, Drüsenschwellungen und
Entzündungen des Rachenraumes, innerlich auch als Laxans, Antidiarrhoikum und zur Behandlung von Magen- und Darmentzündungen verwendet.

Schleime sind Heteropolysaccharide mit Molekulargewichten von ca. 50.000 bis 2.000.000, die durch Extraktion mit heißem oder kaltem Wasser aus der Droge gewonnen werden. Die hochviskosen Lösungen sind im Gegensatz zu den Gummen nicht klebrig. Man unterscheidet saure und neutrale Schleime. Je nach ihrer Zuckerzusammensetzung lassen sie sich in Glucomannane oder Mannane, in Galactomannane, Xylane oder Rhamnogalacturonane einteilen. Nach ihrer Lokalisierung in der Pflanze kann man Vakuolenschleime und Membranschleime unterscheiden. Als Prototyp für einen sauren Pflanzenschleim kann der Schleim der Eibischwurzel angesehen werden. Er enthält L-Rhamnose, D-Galactose, D-Galacturonsäure und D-Glucuronsäure im molaren Verhältnis von ungefähr 3:2:3:3. Der am einfachsten gebaute Teil des Polysaccharids besteht aus sich wiederholenden Undecasaccharid-Untereinheiten.

Für weitere Einzelheiten zu Schleimdrogen und deren Zubereitungen, Wirkungen und Anwendung kann verwiesen werden auf H. Wagner "Pharmazeutische Biologie - Drogen und ihre Inhaltsstoffe", Gustav Fischer Verlag Stuttgart/New York, 1985, insbesondere Seiten 280 ff.

Isländisches Moos ist der deutsche Name für *Lichen islandicus* oder *Cetraria islandica* (*Parmeliaceae*), einer Flechte - entgegen dem deutschen Namen kein Moos -, die aus nördlichen Ländern, wie beispielsweise von Island, Norwegen und Schweden, exportiert wird. Isländisches Moos im eigentlichen Sinne besteht aus dem getrockneten Thallus von *Cetraria islandica* sowie dessen Zubereitungen. Die Droge enthält unter anderem Schleimstoffe und Bitterstoffe sowie bitterschmeckende Flechtensäuren. Aus der gepulverten Flechte lösen sich etwa 60 Gew.-% beim Kochen mit stark verdünnter Natriumhydrogencarbonatlösung, und beim Abkühlen der Lösung entsteht eine Gallerte. Der Extrakt besteht aus einem Polysaccharidgemisch von Lichenin und Isolichenin, einer Reihe von bitterschmeckenden Flechtensäuren (Fumarprotocetrar-, Protocetrar- und Cetrarsäure) sowie Protolichesterinsäure, die sich bei der Aufarbeitung in Lichesterinsäure umwandelt, und Usninsäure als antibiotisch wirkendem Flechtenfarbstoff. Als Schleimdroge besitzt Isländisches Moos reizlindernde Eigenschaften, es wirkt ebenso antimikrobiell. Der Bitterstoffgehalt begründet seine Anwendung bei Appetitlosigkeit. Isländisches Moos wird beispielsweise bei Katarrhen und Durchfall, als Bittertonikum, äußerlich bei schlecht heilenden Wunden, bei kosmetischen Präparaten, in Haarfixiermitteln, als Zusatz zu Biskuitmehl und dergleichen. Das Kaltmazerat und andere bitterschmeckende Zubereitungen der Droge werden zur Behebung der Sub- oder Antacidität eingesetzt. Als Darreichungsformen werden je nach Anwendungsgebiet die zerkleinerte Droge für Aufgüsse sowie andere galenische Zubereitungen bzw. die zerkleinerte Droge vorzugsweise für Kaltmazerate sowie andere bitterschmeckende Zubereitungen zum Einnehmen angewandt. Für weitergehende Einzelheiten zu Isländischem Moos kann beispielsweise auf die folgende Literaturstellen verwiesen werden: Römpp Chemie-Lexikon, 9. Auflage, Band 3, Georg Thieme Verlag, Stuttgart/New York, 1990, Seiten 2055/2056, Stichwort: "Isländisches Moos"; HagerROM 2001, Springer Verlag, Heidelberg, Stichworte: "Cetraria", "Cetraria ericetorum OPIZ", "Cetraria islandica (L.) ACHARIUS" und "Lichen islandicus (Isländisches Moos)"; Monographie "Lichen islandicus (Isländisches Moos)", Bundesanzeiger Nr. 43 vom 2. März 1989; H. Wagner, "Pharmazeutische Biologie - Drogen und ihre Inhaltsstoffe", Gustav Fischer Verlag, Stuttgart/New York, 1985, Seite 281, Stichwort: "Cetrariae Lichen (= Lichen islandicus - Isländisches Moos)".

Die Malve (*Malva sylvestris L.* und *M. neglecta WALLR.*), insbesondere eingesetzt in Form von Malvenblüten und -blättern, ist in Europa, Kleinasien, im Mittelmeergebiet und in Vorderindien heimisch. Zur Anwendung kommen insbesondere die zur Blütezeit gesammelten, rosa-violett gefärbten Blüten bzw. die durch das Trocknen stark eingeschrumpften, gefalteten Blätter, die zumeist in Paketform in den Handel kommen. Die Malve ist ein zwei- bis mehrjähriges Kraut. Der Schleimgehalt von Blüten und Blättern liegt bei 6 bis 8 %. Bei der Hydrolyse liefert der Schleim Glucose, Arabinose, Rhamnose und Galactose. Malvenblüten werden zu Gurgelwässern und Bädern und zusammen mit den Blättern in Teemischungen als Expektorans verwendet.

Im Rahmen der pharmazeutischen Zubereitung nach der vorliegenden Erfindung kann das Isländische Moos und/oder die Malve in Form der Droge vorliegen, insbesondere in Form von zerkleinerten oder pulverisierten Pflanzenteilen oder Pflanzenbestandteilen zugesetzt sein.

Erfindungsgemäß bevorzugt ist es jedoch, wenn das Isländische Moos und/oder die Malve in Form eines Extraktes, insbesondere in Form eines Flüssig- oder Trockenextraktes, vorzugsweise in Form eines Flüssigextraktes, zugesetzt wird; im Fall des Flüssigextraktes kann ein wäßriger, alkoholischer oder wäßrig-alkoholischer Extrakt zugesetzt sein. Auf diese Weise lassen sich besonders hohe Wirkstoffkonzentrationen erreichen.

Wenn die beiden Schleimdrogen Isländisches Moos und Malve in Form eines Extraktes zugesetzt sind, wird vorteilhafterweise jeweils ein Droge/Extrakt-Verhältnis im Bereich von 0,4 : 1 bis 15 : 1, insbesondere 0,5 : 1 bis 10 : 1, bevorzugt 0,5 : 1 bis 5 : 1, besonders bevorzugt etwa 1:1, verwendet.

Die Menge an Isländischem Moos bzw. vorzugsweise dessen Extrakt in der erfindungsgemäßen pharmazeutischen Zubereitung kann in weiten Bereichen variieren. Im allgemeinen liegt sie im Bereich von 0,5 bis 20 Gew.-%, insbesondere 1 bis 10 Gew.-%, bevorzugt 2 bis 7,5 Gew.-%, besonders bevorzugt 3 bis 5 Gew.-%, bezogen auf die pharmazeutische Zubereitung. Dennoch kann es anwendungsbedingt gegebenenfalls vorteilhaft oder erforderlich sein, von den vorgenannten Mengen abzuweichen.

Was die Menge an Malve bzw. vorzugsweise deren Extrakt in der pharmazeutischen Zubereitung anbelangt, so kann deren mengenmäßiger Anteil gleichermaßen in weiten Bereichen variieren. Im allgemeinen liegt die Menge an Malve bzw. vorzugsweise deren Extrakt im Bereich von 0,5 bis 20 Gew.-%, insbesondere 1 bis 10 Gew.%, bevorzugt 2 bis 7,5 Gew.-%, besonders bevorzugt 3 bis 5 Gew.-%, bezogen auf die pharmazeutische Zubereitung.

Im Rahmen der erfindungsgemäßen Kombination bzw. pharmazeutischen Zubereitung wirken die Schleimdrogen Isländisches Moos einerseits und Malve andererseits besonders reizlindernd, insbesondere in Zusammenwirkung mit den übrigen Bestandteilen, da das Isländische Moos und auch die Malve benetzende Stoffe enthalten, welche die angegriffenen Schleimhäute des Mund-/ Rachenraums benetzen und sozusagen einhüllen, die Schleimhäute auf diese Weise vor störenden äußeren Reizen abschirmen und dazu beitragen, daß die natürliche Schutzfunktion der Schleimhäute im Mund- und Rachenraum unterstützt wird. Die pharmazeutische Zubereitung nach der vorliegenden Erfindung in Form einer flüssigen Dosierung ist bei ihrer Applikation sozusagen wie ein "innerer Handschuh" in der Mündhöhle bzw. im Mund-/Rachenraum anzusehen.

Aufgrund ihrer relativ langen Verweildauer im Mund- und Rachenraum sorgt die erfindungsgemäße Zubereitung außerdem für die natürliche Anregung des Speichelflusses, welcher dadurch wiederum zu einer Befeuchtung der ausgetrockneten Schleimhäute führt. Dadurch werden vielfältige Störfaktoren, die zu Schleimhautreizungen führen, beseitigt, und es kommt zu einer wohltuenden Befeuchtung der Mund- und Rachenschleimhaut.

Die erfindungsgemäße pharmazeutische Zubereitung wirkt aber nicht nur reizlindernd und befeuchtend, sondern darüber hinaus auch bakterizid und antimikrobiell. Deshalb ist sie für die zuvor genannten Indikationen besonders geeignet.

Wie zuvor beschrieben, enthält die erfindungsgemäße pharmazeutische Zubereitung aufgrund des Vorhandenseins der beiden Schleimdrogen Isländisches Moos und Malve bzw. deren Extrakten Schleimstoffe, welche eine physikalische Abdeckung der Schleimhaut ergeben und über diese Barriere den darunterliegenden Zellen die Möglichkeit geben, sich zu regenerieren. Diese Schleimhautbarriere hat keine Verbindung chemischer Art mit den Zellen, sondern beruht nur auf einer physikalischen Wirkung. Deshalb ist die erfindungsgemäße pharmazeutische Zubereitung besonders gut verträglich, insbesondere auch für Kinder oder aber für Schwangere und stillende Personen. Aufgrund der Tatsache, daß die Anwendung der erfindungsgemäßen pharmazeutischen Zubereitung mit keinerlei Nebenwirkungen verbunden ist, ist die erfindungsgemäße pharmazeutische Zubereitung insbesondere zur Behandlung der vorgenannten Patienten- bzw. Personengruppen geeignet.

Wie vorstehend geschildert, wird durch die Inhaltsstoffe der erfindungsgemäß eingesetzten Schleimdrogenkombination von Isländischem Moos einerseits und Malve andererseits eine Schutzschicht auf der Schleimhaut des Mund-/ Rachenraums ausgebildet, wodurch Reizeinwirkungen von den Schleimhäuten ferngehalten werden und die Entstehung von Husten gehemmt wird. Auf diese Weise eignet sich die erfindungsgemäße pharmazeutische Zubereitung nicht nur zur prophylaktischen und/oder therapeutischen Behandlung von Schleimhautreizungen und Schleimhautläsionen im Mund- und Rachenraum sowie von Austrocknungen der Schleimhäute im Bereich des Mund- und Rachenraums, sondern insbesondere auch von Husten und Hustenreiz, insbesondere von trockenem Reizhusten. Die erfindungsgemäße pharmazeutische Zubereitung kann insbesondere in Form eines sogenannten Hustensaftes zur Linderung von Hustenreiz bei Erkältungskrankheiten der Atemwege zur Bekämpfung dieses lästigen Hustenreizes eingesetzt werden, ohne daß der natürliche und sinnvolle Hustenreflex unterdrückt wird. Darüber hinaus eignet sich die erfindungsgemäße pharmazeutische Zusammensetzung auch zur prophylaktischen und/oder therapeutischen Behandlung von Heiserkeit sowie von Bronchialkatharren und Bronchialasthma, in den beiden letztgenannten Fällen insbesondere zur unterstützenden Anwendung bzw. Therapie.

Die Anmelderin hat überraschend herausgefunden, daß sich die Wirksamkeit der erfindungsgemäßen pharmazeutischen Zubereitung noch dadurch steigern läßt, daß man außerdem mindestens eine Zinkverbindung, insbesondere mindestens ein Zinksalz, vorzugsweise Zinkgluconat oder Zinksulfat, besonders bevorzugt Zinkgluconat, vorsieht, welches in die pharmazeutische Zubereitung nach der vorliegenden Erfindung inkorporiert bzw. eingearbeitet wird.

Die Zinkverbindung hilft den betroffenen Zellen, freie Radikale zu neutralisieren, und unterstützt damit sinnvoll die zuvor geschilderten physikalischen Effekte von Isländischem Moos und Malve. Auf diese Weise wird die irritierte Schleimhaut im Mund- und Rachenbereich mehrfach gegen Erreger und damit einhergehende freie Radikale geschützt.

Die Menge an Zinkverbindung(en) kann in weiten Bereichen variieren. Im allgemeinen liegt sie im Bereich von 0,001 bis 1 Gew.-%, insbesondere 0,05 bis 0,1 Gew.-%, bezogen auf die pharmazeutische Zubereitung nach der vorliegenden Erfindung. Dennoch kann es anwendungsbedingt gegebenenfalls vorteilhaft oder erforderlich sein, von den vorgenannten Mengen abzuweichen.

Neben den vorgenannten Wirk- und/oder Inhaltsstoffen kann die erfindungsgemäße pharmazeutische Zubereitung außerdem noch weitere Wirk- und/oder Inhaltsstoffe enthalten, insbesondere Verarbeitungshilfsmittel, Aromastoffe, Geschmacksstoffe, Süßstoffe und Süßungsmittel, Säuerungsmittel, Stabilisatoren und/oder Antiseptika.

Im allgemeinen enthält die erfindungsgemäße pharmazeutische Zubereitung insbesondere Zucker und/oder Zuckeraustauschstoffe. Die Menge an Zucker und/oder Zuckeraustauschstoffen kann in weiten Bereichen variieren; insbesondere beträgt die Menge an Zucker und/oder Zuckeraustauschstoffen, bezogen auf die pharmazeutische Zubereitung, 20 bis 80 Gew.-%, insbesondere 20 bis 60 Gew.-%, vorzugsweise 20 bis 50 Gew.-%, wobei es jedoch anwendungsbedingt gegebenenfalls vorteilhaft oder erforderlich sein kann, von den vorgenannten Mengen abzuweichen.

Im Rahmen der erfindungsgemäßen pharmazeutischen Zubereitungen können Zucker und/oder Zuckeraustauschstoffe aller Art eingesetzt werden. Beispiele für erfindungsgemäß geeignete Zucker sind beispielsweise Saccharose, Glucose, insbesondere Dextrose, und Fructose. Die erfindungsgemäß geeigneten Zuckeraustauschstoffe sind insbesondere ausgewählt aus Zuckeralkoholen. Erfindungsgemäß bevorzugte Zuckeraustauschstoffe sind ausgewählt aus der Gruppe von Mannit, Xylit, Sorbit, Isomalt, Maltitsirup, Lactit, Leucrose, Fructoligosacchariden, Glucanen und Polyglucose.

Während Zucker und Süßstoffe gemeinsam als Süßungsmittel bezeichnet werden, werden - im Gegensatz zu den intensiv schmeckenden Süßstoffen - Zuckeraustauschstoffe technologisch wie Saccharose eingesetzt, d. h. sie besitzen einen "Körper" und einen physiologischen Brennwert ("nutritive Zukkeraustauschstoffe"). Die Süßkraft entspricht in weiten Grenzen etwa der von Saccharose. Der physiologische Vorteil der Zuckeraustauschstoffe im Vergleich zu Saccharose liegt in der insulinunhabhängigen Metabolisierung (vorteilhaft z. B. für Diabetiker) und in der zum Teil verminderten kariogenen Wirkung. Für einige Zuckeraustauschstoffe (z. B. Xylit) ist eine antikariogene Wirkung beschrieben.

Der Begriff "Zuckeralkohol" ist die Gruppenbezeichnung für die aus Monosacchariden durch Reduktion der Carbonylgruppe entstehenden Polyhydroxyverbindungen, die keine Zucker sind, gleichwohl aber süß schmecken und deshalb gegebenenfalls Verwendung als Zuckeraustauschstoffe finden können. Man unterscheidet bei diesen im allgemeinen kristallinen, wasserlöslichen Polyolen nach der Anzahl der im Molekül enthaltenen Hydroxygruppen sogenannte Tetrite, Pentite, Hexite usw. Natürlich vorkommende Zuckeralkohole sind z. B. Glycerin, Threit und Erythrit, Adonit (Ribit), Arabit (früher: Lyxit) und Xylit, Dulcit (Galactit), Mannit und Sorbit (Glucit).

Für weitergehende Einzelheiten zu den Begriffen "Zucker", "Zuckeraustauschstoffe" und "Zuckeralkohole" kann beispielsweise verwiesen werden auf Römpp Chemie-Lexikon, 10. Auflage, Band 6, Georg Thieme Verlag, Stuttgart/New York, 1999, Seiten 5096 bis 5100, Stichworte: "Zucker", "Zukkeralkohole" und "Zuckeraustauschstoffe".

Im allgemeinen enthält die pharmazeutische Zubereitung nach der vorliegenden Erfindung die Wirk- und/oder Inhaltsstoffe in einer flüssigen Matrix bzw. Masse als pharmakologisch unbedenklichem Träger, vorzugsweise auf Basis von Wasser und/oder Alkoholen (z. B. Ethanol).

Im allgemeinen enthält die pharmazeutische Zubereitung nach der vorliegenden Erfindung Wasser und/oder Alkohol (z. B. Ethanol), vorzugsweise Wasser, in Mengen von 30 bis 90 Gew.%, insbesondere 35 bis 70 Gew.-%, bezogen auf die pharmazeutische Zubereitung. Insbesondere zur besseren bzw. nebenwirkungsfreien Anwendbarkeit im Fall der Behandlung von Kindern, Stillenden und Schwangeren empfehlt es sich, die pharmazeutische Zubereitung ohne Alkohol, d. h. nur auf der Basis von Wasser, zu formulieren.

Gemäß einer bevorzugten Ausführungsform enthält die pharmazeutische Zubereitung nach der vorliegenden Erfindung in Form einer flüssigen Dosierung, insbesondere zur Anwendung als reizlinderndes Hustenmittel, jeweils bezogen auf die pharmazeutische Zubereitung,
- 0,5 bis 20 Gew.-%, insbesondere 1 bis 10 Gew.-%, vorzugsweise 2 bis 7,5 Gew.-%, besonders bevorzugt 3 bis 5 Gew.-%, Isländisches Moos, bevorzugt in Form von dessen vorzugsweise wäßrigem Extrakt, insbesondere mit einem Droge/Extrakt-Verhältnis im Bereich von 0,5 : 1 bis 5 : 1,
- 0,5 bis 20 Gew.-%, insbesondere 1 bis 10 Gew.-%, vorzugsweise 2 bis 7,5 Gew.-%, besonders bevorzugt 3 bis 5 Gew.-%, Malve, bevorzugt in Form von deren vorzugsweise wäßrigem Extrakt, insbesondere mit einem Droge/Extrakt-Verhältnis im Bereich von 0,5 : 1 bis 5 : 1,
- gegebenenfalls 0,001 bis 1 Gew.-%, insbesondere 0,05 bis 0,1 Gew.- %, mindestens einer Zinkverbindung, insbesondere mindestens eines Zinksalzes, vorzugsweise Zinkgluconat oder Zinksulfat, besonders bevorzugt Zinkgluconat,
- gegebenenfalls 20 bis 80 Gew.-%, insbesondere 20 bis 60 Gew.-%, vorzugsweise 20 bis 50 Gew.-%, Zucker und/oder Zuckeraustauschstoffe,
- 30 bis 90 Gew.-%, insbesondere 35 bis 70 Gew.-%, Wasser und/oder Alkohol, vorzugsweise Wasser.

Die pharmazeutische Zubereitung nach der vorliegenden Erfindung eignet sich, wie vorstehend beschrieben, zur topischen und/oder peroralen Anwendung, vorzugsweise im Mund- und Rachenraum.

Wie zuvor beschrieben, eignet sich die pharmazeutische Zubereitung nach der vorliegenden Erfindung insbesondere zur prophylaktischen und/oder therapeutischen Behandlung von Husten und Hustenreiz, insbesondere trockenem Reizhusten; Schleimhautreizungen und Schleimhautläsionen im Mund- und Rachenraum; Austrocknungen der Schleimhäute im Bereich des Mund- und Rachenraums; Heiserkeit; Bronchialkatharren und Bronchialasthma.

Wie zuvor beschrieben, wird die erfmdungsgemäße pharmazeutische Zubereitung im allgemeinen in Form einer flüssigen Dosierung, insbesondere in Form eines Sirups, appliziert. Eine hochviskose Darreichungsform in Form eines Sirups hat insbesondere den Vorteil, daß die Wirk- und/oder Inhaltsstoffe möglichst lange in Kontakt mit den Schleimhäuten stehen können.

Die Herstellung der erfindungsgemäßen pharmazeutischen Zubereitung kann in an sich bekannter Art und Weise erfolgen. Für weitergehende Einzelheiten kann auf die nachfolgenden Ausführungsbeispiele verwiesen werden. Insbesondere werden zu Zwecken der Herstellung der erfindungsgemäßen pharmazeutischen Zubereitung die einzelnen Wirk- und/oder Inhaltsstoffe in geeigneten Vorrichtungen homogen miteinander zu der anwendungsfertigen Applikationsform vermischt.

Gegenstand der vorliegenden Erfindung ist auch die Verwendung der erfindungsgemäßen pharmazeutischen Zubereitung, wie zuvor beschrieben, zur prophylaktischen und/oder therapeutischen Behandlung von bzw. zur Herstellung eines Arzneimittels zur prophylaktischen und/oder therapeutischen Behandlung von Husten und Hustenreiz, insbesondere trockenem Reizhusten; Schleimhautreizungen und Schleimhautläsionen im Mund- und Rachenraum; Austrocknungen der Schleimhäute im Bereich des Mund- und Rachenraums; Heiserkeit; Bronchialkatharren und Bronchialasthma.

Für die bestimmungsgemäße Verwendung wird die pharmazeutische Zubereitung im allgemeinen in einer Tagesdosis von 30 bis 80 ml, vorzugsweise 40 bis 50 ml, bzw. von 35 bis 96 g, vorzugsweise 45 bis 60 g, verabreicht.

Weiterer Gegenstand der vorliegenden Erfindung ist die Verwendung einer Kombination von Isländischem Moos und Malve, vorzugsweise in Form von deren Extrakten, gegebenenfalls zusammen mit mindestens einer Zinkverbindung, wie zuvor definiert, zur prophylaktischen und/oder therapeutischen Behandlung von bzw. zur Herstellung eines Arzneimittels zur prophylaktischen und/oder therapeutischen Behandlung von Husten und Hustenreiz, insbesondere trockenem Reizhusten; Schleimhautreizungen und Schleimhautläsionen im Mund- und Rachenraum; Austrocknungen der Schleimhäute im Bereich des Mund- und Rachenraums; Heiserkeit; Bronchialkatharren und Bronchialasthma.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die folgenden Ausführungsbeispiele dienen lediglich der Veranschaulichung der vorliegenden Erfindung, ohne sie jedoch hierauf zu beschränken.

### Ausführungsbeispiele:

### Herstellungsbeispiel:

Eine pharmazeutische Zubereitung nach der vorliegenden Erfindung in Form einer flüssigen Dosierung wird ausgehend von den folgenden Bestandteilen bzw. Wirk- und/oder Inhaltsstoffen hergestellt:
100 ml eines erfindungsgemäßen Sirups enthalten:

| | |
|---|---|
| Isländisches Moos (Extrakt 1 : 1) | 5,56 g |
| Malvenblütenextrakt (1 : 1) | 5,56 g |
| Zinkgluconat (entspr. 5 mg Zn/45 ml) | 0,0774 g |
| Kaliumsorbat | 0,241 g |
| Maltitol-Lösung (50 % D-Maltitol) | 58,0 bis 73,0 g |
| Hustenkräuteraroma | 0,60 g |
| gereinigtes Wasser | 50,0 bis 35,0 g |
| gesamt ca. | 120,000 g |

Die Dichte des erfindungsgemäßen Sirups bei 20 °C beträgt ca.1,205 g/ml. Die empfohlene Tagesdosis liegt beispielsweise bei etwa 45 ml bzw. 54 g.
In gleicher Weise werden zwei Vergleichszubereitungen formuliert, wobei die erste Vergleichszubereitung (Vergleichszubereitung I) nur den Extrakt von Isländischem Moos in den vorgenannten Mengen enthält (d. h. die Anteile an Malvenblütenextrakt und Zinkgluconat sind durch einen entsprechenden Anteil an gereinigtem Wasser ersetzt) und die zweite Vergleichszubereitung (Vergleichszubereitung II) nur Malvenblütenextrakt in den vorgenannten Mengen enthält (d. h. die Anteile an Extrakt von Isländischem Moos und Zinkgluconat sind durch einen entsprechenden Anteil an gereinigtem Wasser ersetzt).

### Anwendungsbeispiel:

Zwanzig Patienten mit trockenem Reizhusten und Heiserkeit infolge von Erkältungserkrankungen, einhergehend mit einer Irritation der Schleimhäute im Mund- und Rachenbereich, wurden mit den im vorangehenden Herstellungsbeispiel beschriebenen pharmazeutischen Zubereitungen in Form der zuvor beschriebenen Sirupe therapiert, wobei jeweils eine Gesamttagesdosis von 45 ml bzw. 54 g durch dreifache Gabe jeweils gleicher Mengen verabreicht wurde.

Bei zehn Patienten wurde die erfindungsgemäße pharmazeutische Zubereitung, wie im vorhergehenden Herstellungsbeispiel beschrieben, eingesetzt, während bei fünf der übrigen zehn Patienten die Vergleichszubereitung I und bei den verbleibenden fünf der übrigen zehn Patienten die Vergleichszubereitung II zur Anwendung kam.

Bei einer siebentätigen Anwendung der jeweiligen pharmazeutischen Zubereitungen konnte mit der erfindungsgemäßen pharmazeutischen Zubereitung der Reizhusten und die Heiserkeit sowie die Schleimhautirritationen im Mund-/Rachenraum vollständig austherapiert wurden, während bei den übrigen Patienten, die mit den beiden Vergleichszubereitungen therapiert wurden, zwar eine gewisse Linderung der Symptome beobachtet werden konnte, jedoch nicht deren vollständiges Verschwinden; vielmehr trat eine Austherapierung erst nach mehr als zehn Tagen ein.

Die Untersuchungen zeigen, daß die Anwendung der erfindungsgemäßen Zubereitung in Form einer Kombination von Extrakten von Isländischem Moos und Malve zusammen mit Zinkgluconat einen deutlich gesteigerten Therapieerfolg bewirkt, da durch die erfindungsgemäße Zubereitung ein verbesserter Therapieerfolg - Verschwinden der Symptome - erreicht wird.

Die erfindungsgemäße pharmazeutische Zubereitung führt bei der Behandlung von trockenem Reizhusten und Heiserkeit, einhergehend mit einer Irritation der Schleimhäute des Mund-/Rachenraums, zu einer eindrucksvollen Besserung der hiermit einhergehenden Symptome. Hierin liegt ein entscheidender Vorteil der erfindungsgemäßen Kombination.

## Patentansprüche

1. Pharmazeutische Zubereitung in Form einer flüssigen Dosierung zur peroralen topischen Anwendung als reizlinderndes Hustenmittel, enthaltend eine Kombination von Isländischem Moos (*Lichen islandicus*) und Malve (*Malva sylvestris L.* oder *Malva neglecta WALLR.*) jeweils in Form von deren Extrakten sowie außerdem mindestens eine Zinkverbindung zusammen mit einem pharmakologisch unbedenklichen Träger, wobei die pharmazeutische Zubereitung
- 0,5 bis 20 Gew.-% Isländisches Moos in Form von dessen Extrakt mit einem Droge/Extrakt-Verhältnis im Bereich von 0,4 : 1 bis 15 : 1,
- 0,5 bis 20 Gew.-% Malve in Form von deren Extrakt mit einem Droge/Extrakt-Verhältnis im Bereich von 0,4 : 1 bis 15 : 1 und
- 0,001 bis 1 Gew.-% Zinkverbindung
enthält, wobei alle vorgenannten Gewichtsprozentangaben auf die pharmazeutische Zubereitung bezogen sind.

2. Pharmazeutische Zubereitung nach Anspruch 1, wobei das Isländische Moos und/oder die Malve in Form eines Flüssig- oder Trockenextraktes, vorzugsweise eines Flüssigextraktes, zugesetzt ist.

3. Pharmazeutische Zubereitung nach Anspruch 2, wobei im Fall des Flüssigextrakts ein wäßriger, alkoholischer oder wäßrig-alkoholischer Extrakt zugesetzt ist.

4. Pharmazeutische Zubereitung nach einem oder mehreren der vorangehenden Ansprüche, wobei das Isländische Moos und/oder die Malve in Form eines Extraktes mit einem Droge/Extrakt-Verhältnis im Bereich von 0,5 ; 1 bis 10 : 1, bevorzugt im Bereich von 0,5 : 1 bis 5 : 1, besonders bevorzugt etwa 1 : 1, zugesetzt ist.

5. Pharmazeutische Zubereitung nach einem oder mehreren der vorangehenden Ansprüche, enthaltend Isländisches Moos in Form von dessen Extrakt in Mengen von 1 bis 10 Gew.-%, bevorzugt 2 bis 7,5 Gew.-%, besonders bevorzugt 3 bis 5 Gew.-%, bezogen auf die pharmazeutische Zubereitung.

6. Pharmazeutische Zubereitung nach einem oder mehreren der vorangehenden Ansprüche, enthaltend Malve in Form von deren Extrakt,- in Mengen von 1 bis 10 Gew.-%, bevorzugt 2 bis 7,5 Gew.-%, besonders bevorzugt 3 bis 5 Gew.-%, bezogen auf die pharmazeutische Zubereitung.

7. Pharmazeutische Zubereitung nach einem oder mehreren der vorangehenden Ansprüche, enthaltend die Zinkverbindung, insbesondere in Form eines Zinksalzes, vorzugsweise in Form von Zinkgluconat oder Zinksulfat, besonders bevorzugt Zinkgluconat, in Mengen von 0,05 bis 0,1 Gew.-%, bezogen auf die pharmazeutische Zubereitung.

8. Pharmazeutische Zubereitung nach einem oder mehreren der vorangehenden Ansprüche, enthaltend außerdem weitere Wirk- und/oder Inhaltsstoffe, insbesondere ausgewählt aus der Gruppe von Verarbeitungshilfsmitteln, Aromastoffen, Geschmacksstoffen, Süßstoffen und Süßungsmitteln, Säuerungsmittel, Stabilisatoren und/oder Antiseptika.

9. Pharmazeutische Zubereitung nach einem oder mehreren der vorangehenden Ansprüche, enthaltend Zucker und/oder Zuckeraustauschstoffe, insbesondere in Mengen von 20 bis 80 Gew.-%, insbesondere 20 bis 60 Gew.-%, vorzugsweise 20 bis 50 Gew.-%, bezogen auf die pharmazeutische Zubereitung.

10. Pharmazeutische Zubereitung nach einem oder mehreren der vorangehenden Ansprüche, enthaltend die Wirk- und/oder Inhaltsstoffe in einer flüssigen Matrix und/oder Masse als pharmakologisch unbedenklichem Träger, vorzugsweise auf Basis von Wasser und/oder Alkoholen, insbesondere Ethanol, besonders bevorzugt auf der Basis von Wasser.

11. Pharmazeutische Zubereitung nach einem oder mehreren der vorangehenden Ansprüche, enthaltend Wasser und/oder Alkohol, insbesondere Ethanol, vorzugsweise Wasser, in Mengen von 30 bis 90 Gew.-%, insbesondere 35 bis 70 Gew.- %, bezogen auf die pharmazeutische Zubereitung.

12. Pharmazeutische Zubereitung nach einem oder mehreren der vorangehenden Ansprüche, enthaltend, jeweils bezogen auf die pharmazeutische Zubereitung,
- 0,5 bis 20 Gew.-% Isländisches Moos in Form von dessen vorzugsweise wäßrigem Extrakt mit einem Droge/Extrakt-Verhältnis im Bereich von 0,5 : 1 bis 5 : 1,
- 0,5 bis 20 Gew.-% Malve in Form von deren vorzugsweise wäßrigem Extrakt mit einem Droge/Extrakt-Verhältnis im Bereich von 0,5 : 1 bis 5 : 1,
- 0,001 bis 1 Gew.-% mindestens einer Zinkverbindung, insbesondere mindestens eines Zinksalzes, vorzugsweise Zinkgluconat oder Zinksulfat, besonders bevorzugt Zinkgluconat,
- gegebenenfalls 20 bis 80 Gew.-% Zucker und/oder Zuckeraustauschstoffe,
- 30 bis 90 Gew.-% Wasser und/oder Alkohol, vorzugsweise Wasser.

13. Pharmazeutische Zubereitung nach einem oder mehreren der vorangehenden Ansprüche zur peroralen topischen Anwendung im Mund- und Rachenraum.

14. Pharmazeutische Zubereitung nach einem oder mehreren der vorangehenden Ansprüche zur prophylaktischen und/oder therapeutischen Behandlung von Husten und Hustenreiz, insbesondere trockenem Reizhusten; Schleimhautreizungen und Schleimhautläsionen im Mund- und Rachenraum; Austrocknungen der Schleimhäute im Bereich des Mund- und Rachenraums; Heiserkeit; Bronchialkatarrhen und Bronchialasthma.

15. Verwendung einer pharmazeutischen Zubereitung nach den vorangehenden Ansprüchen zur Herstellung eines Arzneimittels zur prophylaktischen und/oder therapeutischen Behandlung von Husten und Hustenreiz, insbesondere trockenem Reizhusten; Schleimhautreizungen und Schleimhautläsionen im Mund- und Rachenraum; Austrocknungen der Schleimhäute im Bereich des Mund- und Rachenraums; Heiserkeit; Bronchialkatarrhen und Bronchialasthma.

16. Verwendung nach Anspruch 15, **dadurch gekennzeichnet, daß** die pharmazeutische Zubereitung in einer Tagesdosis von 30 bis 80 ml, vorzugsweise 40 bis 50 ml, verabreicht wird.

## Claims

1. Pharmaceutical preparation in the form of a liquid dosage for peroral topical application as soothing cough remedy comprising a combination of Icelandic moss (*Lichen islandicus*) and mallow (*Malva sylvestris L.* or *Malva neglecta WALLR*) in each case in the form of extracts thereof, and additionally at least one zinc compound together with a pharmacologically acceptable carrier, where the pharmaceutical preparation comprises
- 0.5 to 20 % by weight of Icelandic moss in the form of an extract thereof with an extract ratio in the range from 0.4 : 1 to 15 : 1,
- 0.5 to 20 % by weight of mallow in the form of an extract thereof with an extract ratio in the range from 0.4 : 1 to 15 : 1 and
- 0.001 to 1 % by weight of zinc compound,
where all the aforementioned percent by weight data are based on the pharmaceutical preparation.

2. Pharmaceutical preparation according to Claim 1, where the Icelandic moss and/or the mallow is added in the form of a liquid or dry extract, preferably of a liquid extract.

3. Pharmaceutical preparation according to Claim 2, where in the case of the liquid extract an aqueous, alcoholic or hydroalcoholic extract is added.

4. Pharmaceutical preparation according to one or more of the preceding claims, where the Icelandic moss and/or the mallow is added in the form of an extract with an extract ratio in the range from 0.5 : 1 to 10 : 1, preferably in the range from 0.5 : 1 to 5 : 1, particularly preferably about 1:1.

5. Pharmaceutical preparation according to one or more of the preceding claims, comprising Icelandic moss in the form of an extract thereof in amounts of from 1 to 10 % by weight, preferably 2 to 7.5 % by weight, particularly preferably 3 to 5 % by weight, based on the pharmaceutical preparation.

6. Pharmaceutical preparation according to one or more of the preceding claims, comprising mallow in the form of an extract thereof in amounts of from 1 to 10 % by weight, preferably 2 to 7.5 % by weight, particularly preferably 3 to 5 % by weight, based on the pharmaceutical preparation.

7. Pharmaceutical preparation according to one or more of the preceding claims, comprising the zinc compound, in particular in the form of a zinc salt, preferably in the form of zinc gluconate or zinc sulphate, particularly preferably zinc gluconate, in amounts of from 0.05 to 0.1 % by weight, based on the pharmaceutical preparation.

8. Pharmaceutical preparation according to one or more of the preceding claims, additionally comprising further active substances and/or ingredients, in particular selected from the group of processing aids, aromatizers, flavourings, sweeteners and sweetening agents, acidifiers, stabilizers and/or antiseptics.

9. Pharmaceutical preparation according to one or more of the preceding claims, comprising sugar and/or sugar substitutes, in particular in amounts of from 20 to 80 % by weight, in particular 20 to 60 % by weight, preferably 20 to 50 % by weight, based on the pharmaceutical preparation.

10. Pharmaceutical preparation according to one or more of the preceding claims, comprising the active substances and/or ingredients in a liquid matrix and/or composition as pharmacologically acceptable carrier, preferably based on water and/or alcohols, in particular ethanol, particularly preferably based on water.

11. Pharmaceutical preparation according to one or more of the preceding claims, comprising water and/or alcohol, in particular ethanol, preferably water, in amounts of from 30 to 90 % by weight, in particular 35 to 70 % by weight, based on the pharmaceutical preparation.

12. Pharmaceutical preparation according to one or more of the preceding claims, comprising, in each case based on the pharmaceutical preparation,
- 0.5 to 20 % by weight of Icelandic moss in the form of a preferably aqueous extract thereof with an extract ratio in the range from 0.5 : 1 to 5:1,
- 0.5 to 20 % by weight of mallow in the form of a preferably aqueous extract thereof with an extract ratio in the range from 0.5 : 1 to 5 : 1,
- 0.001 to 1 % by weight of at least one zinc compound, in particular of at least one zinc salt, preferably zinc gluconate or zinc sulphate, particularly preferably zinc gluconate,
- where appropriate 20 to 80 % by weight of sugar and/or sugar substitutes,
- 30 to 90 % by weight of water and/or alcohol, preferably water.

13. Pharmaceutical preparation according to one or more of the preceding claims for peroral topical application in the mouth and throat.

14. Pharmaceutical preparation according to one or more of the preceding claims for the prophylactic and/or therapeutic treatment of cough and the urge to cough, especially dry cough; mucosal irritations and mucosal lesions in the mouth and throat; drying of the mucous membranes in the region of the mouth and throat; hoarseness; bronchial catarrhs and bronchial asthma.

15. Use of a pharmaceutical preparation according to the preceding claims for the manufacture of a medicament for the prophylactic and/or therapeutic treatment of cough and the urge to cough, especially dry cough; mucosal irritations and mucosal lesions in the mouth and throat; drying of the mucous membranes in the region of the mouth and throat; hoarseness; bronchial catarrhs and bronchial asthma.

16. Use according to Claim 15, **characterized in that** the pharmaceutical preparation is administered in a daily dose of from 30 to 80 ml, preferably 40 to 50 ml.

## Revendications

1. Préparation pharmaceutique présentée en une forme galénique liquide destinée à l'administration topique per os en tant qu'antitussif calmant l'irritation, qui contient une association de mousse d'Islande (*Lichen islandicus*) et de mauve sylvestre (*Malva sylvestris L.* ou *Malva neglecta WALLR.*), chacune sous forme de leurs extraits, ainsi qu'en outre au moins un composé contenant du zinc, conjointement avec un véhicule pharmacologiquement acceptable, la préparation pharmaceutique contenant
- de 0,5 à 20 % en poids de mousse d'Islande sous forme de son extrait, à un rapport drogue/extrait dans la plage de 0,4 : 1 à 15 : 1,
- de 0,5 à 20 % en poids de mauve sylvestre sous forme de son extrait, à un rapport drogue/extrait dans la plage de 0,4 : 1 à 15 : 1,
- de 0,001 à 1 % en poids de composé contenant du zinc
tous les pourcentages en poids précités se rapportant à la préparation pharmaceutique.

2. Préparation pharmaceutique selon la revendication 1, dans laquelle la mousse d'Islande et/ou la mauve sylvestre est/sont utilisée(s) sous forme d'un extrait liquide ou sec, de préférence d'un extrait liquide.

3. Préparation pharmaceutique selon la revendication 2, dans laquelle, dans le cas de l'extrait liquide on ajoute un extrait aqueux, alcoolique ou aqueux-alcoolique.

4. Préparation pharmaceutique selon une ou plusieurs des revendications précédentes, dans laquelle on ajoute la mousse d'Islande et/ou la mauve sylvestre sous forme d'un extrait à un rapport drogue/extrait dans la plage de 0,5 : 1 à 10 : 1, de préférence dans la plage de 0,5 : 1 à 5 : 1, de façon particulièrement préférée d'environ 1:1.

5. Préparation pharmaceutique selon une ou plusieurs des revendications précédentes, contenant de la mousse d'Islande, sous forme de son extrait, en quantités de 1 à 10 % en poids, de préférence de 2 à 7,5 % en poids, de façon particulièrement préférée de 3 à 5 % en poids, par rapport à la préparation pharmaceutique.

6. Préparation pharmaceutique selon une ou plusieurs des revendications précédentes, contenant de la mauve sylvestre, sous forme de son extrait, en quantités de 1 à 10 % en poids, de préférence de 2 à 7,5 % en poids, de façon particulièrement préférée de 3 à 5 % en poids, par rapport à la préparation pharmaceutique.

7. Préparation pharmaceutique selon une ou plusieurs des revendications précédentes, qui contient le composé contenant du zinc, en particulier sous forme de sel de zinc, de préférence sous forme de gluconate de zinc ou de sulfate de zinc, de façon particulièrement préférée de gluconate de zinc, en quantités de 0,05 à 0,1 % en poids, par rapport à la préparation pharmaceutique.

8. Préparation pharmaceutique selon une ou plusieurs des revendications précédentes, contenant en outre d'autres principes actifs et/ou composants, en particulier choisis dans l'ensemble constitué par des adjuvants de fabrication, des arômes, des agents de sapidité, des édulcorants et des agents sucrants, des acidifiants, des stabilisants et/ou des antiseptiques.

9. Préparation pharmaceutique selon une ou plusieurs des revendications précédentes, contenant du sucre ou des succédanés de sucre, en particulier en quantités de 20 à 80 % en poids, plus particulièrement de 20 à 60 % en poids, de préférence de 20 à 50 % en poids, par rapport à la préparation pharmaceutique.

10. Préparation pharmaceutique selon une ou plusieurs des revendications précédentes, contenant les principes actifs et/ou les composants dans une matière et/ou matrice liquide en tant que véhicule pharmaceutiquement acceptable, de préférence à base d'eau et/ou d'alcools, en particulier d'éthanol, de façon particulièrement préférée à base d'eau.

11. Préparation pharmaceutique selon une ou plusieurs des revendications précédentes, contenant de l'eau et/ou un alcool, en particulier de l'éthanol, de préférence de l'eau, en quantités de 30 à 90 % en poids, en particulier de 35 à 70 % en poids, par rapport à la préparation pharmaceutique.

12. Préparation pharmaceutique selon une ou plusieurs des revendications précédentes, contenant, chaque fois par rapport à la préparation pharmaceutique,
- de 0,5 à 20 % en poids de mousse d'Islande sous forme de son extrait de préférence aqueux, à un rapport drogue/extrait dans la plage de 0,5 : 1 à 5 : 1,
- de 0,5 à 20 % en poids de mauve sylvestre sous forme de son extrait de préférence aqueux, à un rapport drogue/extrait dans la plage de 0,5 : 1 à 5:1,
- de 0,001 à 1 % en poids d'au moins un composé contenant du zinc, en particulier d'au moins un sel de zinc, de préférence de gluconate de zinc ou de sulfate de zinc, de façon particulièrement préférée de gluconate de zinc,
- éventuellement de 20 à 80 % en poids de sucre et/ou de succédanés de sucre,
- de 30 à 90 % en poids d'eau et/ou d'alcool, de préférence d'eau.

13. Préparation pharmaceutique selon une ou plusieurs des revendications précédentes, destinée à l'administration topique per os dans la cavité buccale et pharyngée.

14. Préparation pharmaceutique selon une ou plusieurs des revendications précédentes, destinée au traitement prophylactique et/ou thérapeutique de la toux et de l'irritation tussigène, en particulier de la toux sèche d'irritation; des irritations des muqueuses et des lésions des muqueuses dans la cavité buccale et pharyngée ; de sécheresses des muqueuses dans la région de la cavité buccale et pharyngée ; de l'enrouement ; de la bronchite et de l'asthme bronchique.

15. Utilisation d'une préparation pharmaceutique selon les revendications précédentes, pour la fabrication d'un médicament destiné au traitement prophylactique et/ou thérapeutique de la toux et de l'irritation tussigène, en particulier de la toux sèche d'irritation; des irritations des muqueuses et des lésions des muqueuses dans la cavité buccale et pharyngée; de sécheresses des muqueuses dans la région de la cavité buccale et pharyngée; de l'enrouement ; de la bronchite et de l'asthme bronchique.

16. Utilisation selon la revendication 15, **caractérisée en ce que** la préparation pharmaceutique est administrée à une dose journalière de 30 à 80 ml, de préférence de 40 à 50 ml.
